Europäisches Patentamt

(19) European Patent Office

Office européen des brevets

(11) Veröffentlichungsnummer: **0 127 103**
**B1**

(12) **EUROPÄISCHE PATENTSCHRIFT**

(45) Veröffentlichungstag der Patentschrift:
28.01.87

(51) Int. Cl.⁴: **A 61 F 13/16**

(21) Anmeldenummer: **84105792.0**

(22) Anmeldetag: **21.05.84**

(54) **Damenbinde.**

(30) Priorität: **28.05.83 DE 3319421**

(43) Veröffentlichungstag der Anmeldung:
**05.12.84 Patentblatt 84/49**

(45) Bekanntmachung des Hinweises auf die Patenterteilung:
**28.01.87 Patentblatt 87/5**

(84) Benannte Vertragsstaaten:
**AT BE CH DE FR GB IT LI LU NL SE**

(56) Entgegenhaltungen:
**EP - A - 0 040 085**
**WO - A - 82/03170**
**DE - U - 8 319 198**
**GB - A - 2 048 684**
**US - A - 3 315 677**
**US - A - 4 285 343**

(73) Patentinhaber: **Vereinigte Papierwerke AG,**
**Schoppershofstrasse 80, D-8500 Nürnberg (DE)**

(72) Erfinder: **Sustmann, Scarlett, Dr., Am**
**Nachtigallenwäldchen 34, D-4060 Viersen 12 (DE)**

(74) Vertreter: **Pohl, Hans Ludwig, Hefnersplatz 3,**
**D-8500 Nürnberg 11 (DE)**

## Beschreibung

Die Erfindung betrifft eine Damenbinde mit undurchlässiger Wäscheschutzfolie auf der körperabgewandten Bindenfläche.

Bekannte Damenbinden unterscheiden sich u.a. im Aufbau der Saugschicht. Wesentlicher Bestandteil der Saugschicht ist meist Holzpulpe. Daneben sind Konstruktionen bekannt, die zusätzlich Tissue-Lagen, Watte und auch hochabsorbierende synthetische Quellmittel, z.B. Polyacrylate, halbsynthetische bzw. gepfropfte Stärkederivate, oder natürliche Quellmittel, z.B. Alginate, enthalten, um das Absorptionsvermögen zu erhöhen. Üblicherweise wird das Saugkissen an der körperabgewandten Seite mit einer undurchlässigen, z.B. aus Polyäthylen oder Polypropylen bestehenden, Folie versehen. Diese Folie wird bei einer Reihe von Marktprodukten bis an die Oberseite der Binde hochgezogen, um ein seitliches Durchdringen von Flüssigkeit zu verhindern.

Die Gesamtkonstruktion der Binde, d.h. die Kombination von Saugkissen und Folie, wird in der Regel mit einem Nonwoven aus Zellwolle, Zellwolle und Kunstfaser, z.B. Polyester oder Polypropylen, oder aus reiner Kunstfaser umhüllt. Schliesslich werden an der vom Körper abgewandten Seite Haftelemente angebracht. Die zum Befestigen im Slip dienenden Haftelemente reichen nicht bis zum äussersten seitlichen Längsrand der Binde, da in diesem Bereich die Gefahr einer Verklebung mit der Haut oder Behaarung besteht.

Obwohl die bekannten Binden einen recht guten Wäscheschutz darstellen, kommt es immer wieder zu Wäschebeschmutzung, wenn sich die Binde beim Tragen faltet und seitlich Flüssigkeit vorbeidringt, weil infolge der Faltung die nicht verklebten Randbezirke der Binde zur Körperseite oder zur körperabgewandten Seite abgebogen werden.

Der Erfindung liegt die Aufgabe zugrunde, die Bindenkonstruktion so zu verbessern, dass ein seitliches Vorbeidringen von Flüssigkeit an der Binde praktisch ausgeschlossen ist. Die erfindungsgemässe Lösung ist für die eingangs genannte Damenbinde mit undurchlässiger Wäscheschutzfolie auf der körperabgewandten Bindenfläche gekennzeichnet durch eine weitere vollflächig auf die körperabgewandte Bindenfläche aufgebrachte, seitlich über die Bindenlängsränder überstehende Schicht aus flüssigkeitsundurchlässigem Wäscheschutzmaterial. Verbesserungen und weitere Ausgestaltungen der Erfindung werden in den abhängigen Ansprüchen beschrieben.

Durch das vollflächige Aufbringen, insbesondere Bekleben, bei deutlichem seitlichem Überstand von insbesondere 1,5 bis 5 cm mit einem undurchlässigen Wäscheschutzmaterial wird erreicht, dass seitlich bzw. an den Längsrändern der Binde Flüssigkeit normalerweise nicht mehr vorbeidringen kann. Das erfindungsgemäss aufzubringende zusätzliche flüssigkeitsundurchlässige Wäscheschutzmaterial kann im einfachsten Fall aus einer herkömmlichen Folie, z.B. aus Polyäthylen oder aus Polypropylen, bestehen. Vorzugsweise wird das zusätzliche Wäscheschutzmaterial jedoch aus einem Verbundmaterial, insbesondere aus einem die flüssigkeitsundurchlässige Folie kaschierenden, hautfreundlichen, weichen Zellwollvlies, hergestellt. Es kann aber auch vorteilhaft sein, zwischen Folie und Vliesstoff eine dünne nur wenige Millimeter dicke Saugmaterialschicht, z.B. ein saugfähiges Vlies oder eine dünne Zellwoll- oder Zellstoffschicht einzufügen.

Dieses — wie auch immer geartete — erfindungsgemäss zusätzlich vorgesehene Wäscheschutzmaterial, dessen Breite grösser als die Breite von Vorlage bzw. Binde sein soll, wird grundsätzlich so angeordnet, dass die Bindenkonstruktion mit ihrer Unterseite auf der hautfreundlichen Vliesschicht aufliegt bzw. aufgeklebt ist und die undurchlässige Folie auf der vom Körper abgewandten Aussenseite liegt. Auf diese Unterseite des erfindungsgemässen zusätzlichen Wäscheschutzes können in üblicher Weise Haftelemente aufgebracht werden.

Gemäss weiterer Erfindung kann der seitliche Überstand des zusätzlichen Wäscheschutzmaterials, insbesondere durch Stanzen, so geformt werden, dass er bequem nach unten um den Schritt-Teil des Slips zu legen ist. Hierzu können auf dem überstehenden Randbereich des Zusatz-Wäscheschutzes ebenfalls Haftelemente angebracht werden. Da zumindest die der Binde zugewandte Fläche des Zusatz-Wäscheschutzes mit einer hautfreundlichen Beschichtung versehen werden soll, kann die gegebenenfalls auf der Rückseite freiliegende Folie nicht mit der Haut in Berührung kommen. Auch eine Gefahr der Verklebung der Haftelemente mit der Haut oder Behaarung besteht nicht.

Anhand der schematischen Darstellung von Ausführungsbeispielen werden weitere Einzelheiten der Erfindung erläutert. Es zeigen:

Fig. 1 einen Querschnitt durch ein als Zusatz-Wäscheschutz geeignetes Verbundmaterial;

Fig. 2 eine Draufsicht auf eine auf der Unterseite mit dem Zusatz-Wäscheschutz beklebte Binde;

Fig. 3 einen zum seitlichen Umlegen um den Schritt-Teil des Slips ausgelegten Zusatz-Wäscheschutz mit aufgebrachter Binde;

Fig. 4 eine Ausbildung der Unterseite des Zusatz-Wäscheschutzes nach Fig. 3;

Fig. 5 einen Slip mit eingelegter Binde; und

Fig. 6 den Schritt-Teil des Slips nach Fig. 5 in der Ansicht von unten.

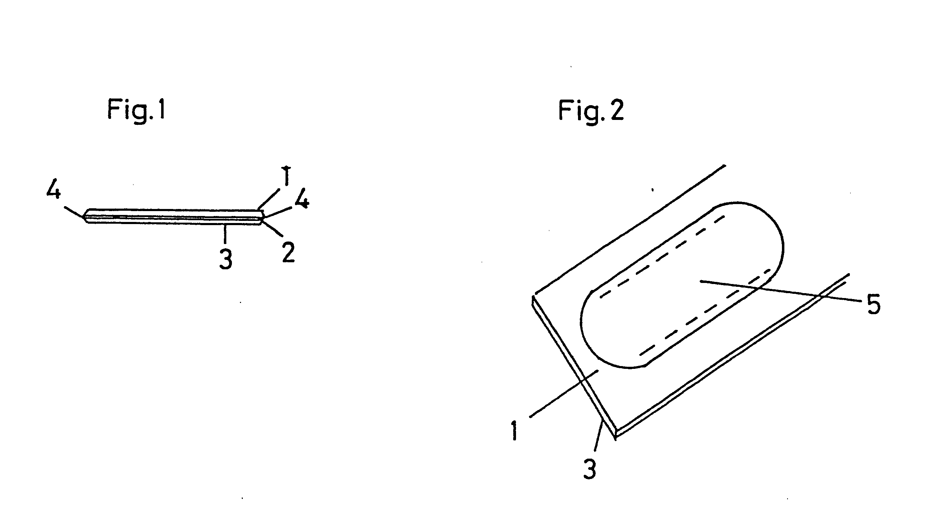

Ein Ausführungsbeispiel eines als zusätzliches Wäscheschutzmaterial zum Aufbringen auf die Unterseite einer Damenbinde vorteilhaften Verbundmaterials wird im Schnitt in Fig. 1 dargestellt. Das Verbundmaterial besteht aus einer Nonwoven-Schicht 1, einer Saugschicht 2 und einer flüssigkeitsundurchlässigen Folie 3. Die Kanten werden vorzugsweise durch Siegelnähte 4 verschlossen. Die Saugschicht 2 soll vorzugsweise eine Schichtdicke von wenigen Millimetern besitzen.

Das Verbundmaterial nach Fig. 1 wird gemäss Fig. 2 so auf die Unterseite einer Damenbinde 5 aufgeklebt, dass die Folie 3 auf der der Binde abgewandten Fläche und die Nonwoven-Schicht 1 auf der der Binde 5 zugewandten Fläche liegt (Fig. 2).

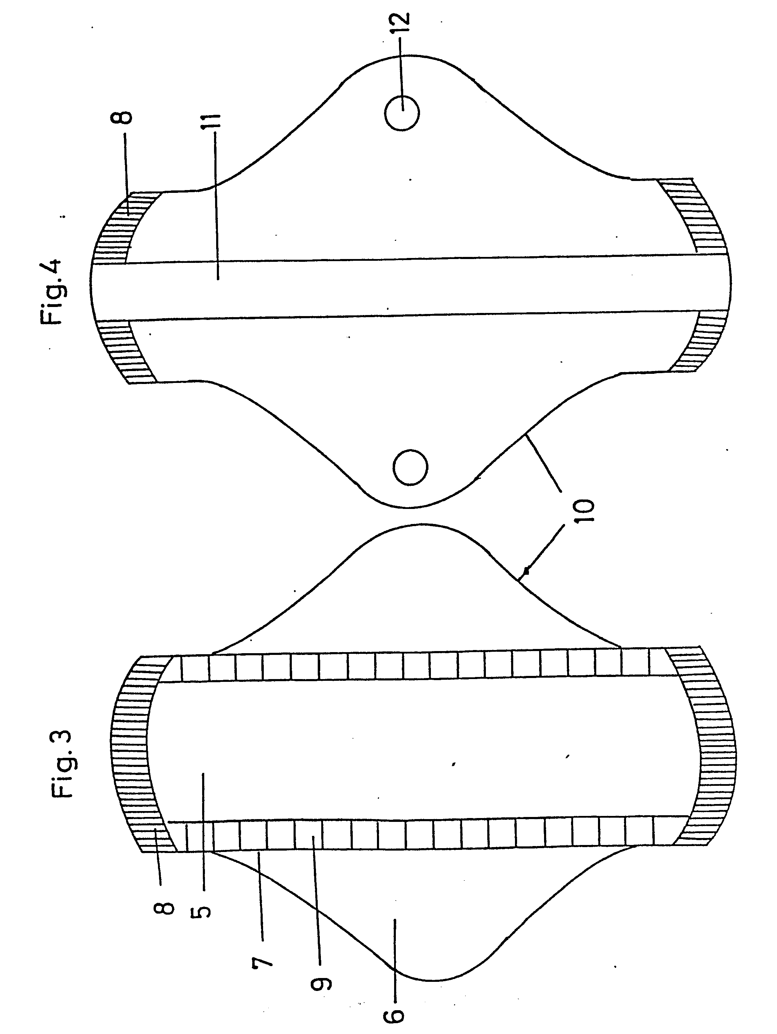

Der grössenordnungsgemäss etwa 1,5 bis 5 cm breite seitliche Überstand des Zusatz-Wäscheschutzes über die Längsränder 7 der Binde 5 kann vorzugsweise so geformt werden, dass er um den Schritt-Teil des jeweiligen Slips zu legen ist. In Fig. 3 wird die dem Körper zuzuwendende Seite des Zusatz-Wäscheschutzes, also dessen Nonwoven-Fläche 1 mit darauf liegender Binde 5 dargestellt. Diese besitzt die übliche Endenprägung bzw. Siegelung 8. Die innere Wäscheschutzfolie 9 der Binde 5 selbst ist bis auf die in Fig. 3 sichtbare Bindenoberseite vorgezogen.

In Fig. 4 wird dargestellt, wie der insgesamt mit 10 bezeichnete Zusatz-Wäscheschutz auf der körperabgewandten Seite in üblicher Weise mit einem Haftstreifen 11 versehen werden kann. Vorzugsweise werden auch an der Unterseite des seitlichen Überstands 6 Haftelemente 12 angebracht, die zum Befestigen an der körperabgewandten Seite des Schritt-Teils des Slips dienen.

Aus der Zeichnung des teilweise im Schnitt dargestellten Slips mit eingelegter Binde ist zu erkennen, in welcher Weise der seitliche Überstand 6 des Zusatz-Wäscheschutzes 10 um den Schritt-Teil 13 aussen herumzulegen und auf der Unterseite mit Hilfe der Haftelemente 12 zu befestigen ist. Aus der Slip-Innenseite liegt die Binde mit Saugkissen 14 und innerer Wäscheschutzfolie 9 sowie gegebenenfalls darauf aufgebrachter Nonwoven-Schicht 15 auf.

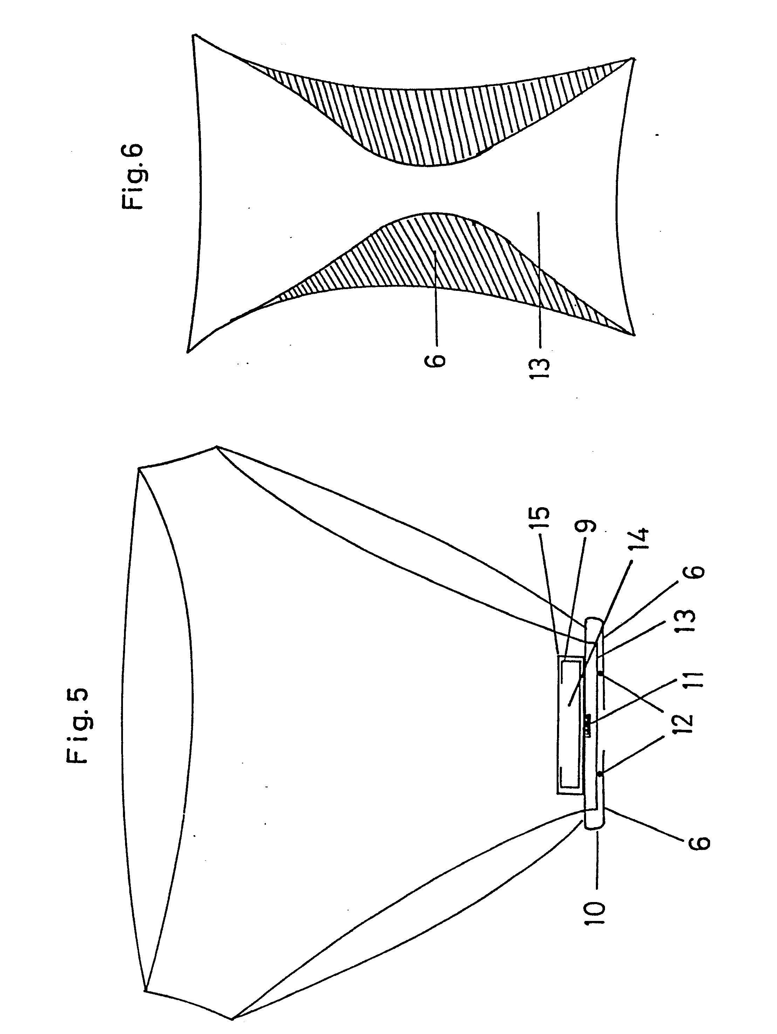

Die Unterseite bzw. Aussenansicht des Schritt-Teils 13 nach Fig. 5 wird in Fig. 6 dargestellt. Ersichtlich ergibt sich durch den Zusatz-Wäscheschutz 10 auch eine weitere Fixierung der Binde gegen Verrutschen an der dem Körper abgewandten Seite des Slips. Schliesslich kommt das Verbundmaterial des Zusatz-Wäscheschutzes 10 nur mit der hautfreundlichen Nonwoven-Fläche 1 mit dem Körper in Berührung.

Die Herstellung der mit dem erfindungsgemässen Zusatz-Wäscheschutz versehenen Binde kann in den Produktionsgang einer üblichen Bindenmaschine integriert werden. Hierbei können die Saugkissen — abgedeckt mit einer flüssigkeitsundurchlässigen Folie und überlappend in Nonwoven eingeschlagen — auf eine Bahn vorgegebener Breite des zusätzlichen Wäscheschutzmaterials auflaufen. Das Prägen der Enden und die Formgebung des Zusatz-Wäscheschutzes kann in einem Arbeitsgang mit einer entsprechenden Stanz-Prägewalze ausgeführt werden. Der dabei entstehende Abfall kann als Recyclingware dem Defibrator zugeführt werden, insbesondere bei Konstruktionen mit zwei Fluffkissen, die aus zwei unabhängigen Defibratoren gebildet werden.

## Patentansprüche

1. Damenbinde (5) mit undurchlässiger Wäscheschutzfolie (9) auf der körperabgewandten Bindenfläche, gekennzeichnet durch eine weitere vollflächig auf die körperabgewandte Bindenfläche aufgebrachte, seitlich über die Bindenlängsränder (7) überstehende Schicht aus flüssigkeitsundurchlässigem Wäscheschutzmaterial (10).

2. Binde nach Anspruch 1, dadurch gekennzeichnet, dass der seitliche Überstand (6) der Schicht des Zusatz-Wäscheschutzmaterials (10) etwa 1,5 bis 5 cm beträgt.

3. Binde nach Anspruch 1 oder 2, welche in einem Slip zu tragen ist, dadurch gekennzeichnet, dass der seitliche Überstand (6) im Hinblick auf ein Umlegen auf die körperabgewandte Seite des Schritt-Teils (13) des Slips geformt ist.

4. Binde nach einem oder mehreren der Ansprüche 1 bis 3, gekennzeichnet durch Haftelemente (12) zum Befestigen des Überstands (6) an der körperabgewandten Seite des Slips.

5. Binde nach einem oder mehreren der Ansprüche 1 bis 4, dadurch gekennzeichnet, dass der Zusatz-Wäscheschutz (10) aus einer Folie, z.B. aus Polyäthylen oder Polypropylen, besteht.

6. Binde nach einem oder mehreren der Ansprüche 1 bis 5, dadurch gekennzeichnet, dass der Zusatz-Wäscheschutz (10) aus einem Verbundmaterial aus einer hautfreundlichen Nonwoven-Schicht (1) oder dergleichen und einer flüssigkeitsundurchlässigen Folie (3) besteht.

7. Binde nach einem oder mehreren der Ansprüche 1 bis 6, dadurch gekennzeichnet, dass der Zusatz-Wäscheschutz (10) aus einer hautfreundlichen Deckschicht (1), einer mittig liegenden Saugschicht (2), vorzugsweise von weniger als 4 mm Dicke, und einer flüssigkeitsundurchlässigen Folie (3) besteht.

8. Binde nach einem oder mehreren der Ansprüche 1 bis 7, dadurch gekennzeichnet, dass der Zusatz-Wäscheschutz (10) mit der hautfreundlichen Oberfläche an der körperabgewandten Seite der Binde anliegt, insbesondere klebt.

9. Binde nach einem oder mehreren der Ansprüche 1 bis 8, dadurch gekennzeichnet, dass der seitliche Überstand (6) an den Längsenden (8) der Binde übersteht.

## Claims

1. Sanitary towel (5) with impermeable laundry protection film (9) on the towel surface remote from the body, characterised by a further layer which is of laundry protection material (10) impermeable by liquid, mounted in full area on the towel surface remote from the body and which is projecting laterally beyond the longitudinal edges (7) of the towel.

2. Towel according to claim 1, characterised thereby, that the lateral projection (6) of the layer of the additional laundry protection material (10) amounts to about 1.5 to 5 centimetres.

3. Towel according to Claim 1 or 2, which is to be worn in a slip, characterised thereby, that the lateral projection (6) is shaped with a view to being folded over onto that side of the step part (13) of the slip, which is remote from the body.

4. Towel according to one or more of the claim 1 to 3, characterised by adhesive elements (12) for the fastening of the projection (6) at that side of the slip, which is remote from the body.

5. Towel according to one or more of the claims 1 to 4, characterised thereby, that the additional laundry protection (10) consists of a film, for example of polyethylene or polypropylene.

6. Towel according to one or more of the claims 1 to 5, characterised thereby, that the additional laundry protection (10) consists of a compound material of a non-woven layer (1) or the like, which is kind to the skin, and a film (3), which is impermeable by liquid.

7. Towel according to one or more of the claims 1 to 6, characterised thereby, that the additional laundry protection (10) consists of a cover layer (1), which is kind to the skin, a centrally lying suction layer (2), preferably less than 4 millimetres in thickness and a film (3), which is impermeably by liquid.

8. Towel according to one or more of the claims 1 to 7, characterised thereby, that the additional laundry protection (10) rests, in particular adheres by the surface, which is kind to the skin, against that side of the towel, which is remote from the body.

9. Towel according to one or more of the claims 1 to 8, characterised thereby, that the lateral projection (6) projects at the longitudinal ends (8) of the towel.

**Revendications**

1. Serviette hygiénique (5) avec feuille imperméable de protection du linge (9) sur la surface de la serviette qui est opposée au corps, caractérisée par le fait qu'elle comporte de la matière suplémentaire de protection du linge (10), imperméable aux liquides, appliquée sur toute la surface de celle de la serviette qui est opposée au corps et dépassant latéralement les bords longitudinaux (7) de celle-ci.

2. Serviette selon la revendication 1, caractérisée par le fait que le dépassement latéral (6) de la matière supplémentaire de protection du linge (10) est d'environ 1,5 à 5 cm.

3. Serviette selon l'une ou l'autre des revendications 1 ou 2, destinée à portée dans un slip, caractérisée par le fait que le dépassement latéral (6) présente la forme voulue pour se replier sur la partie de l'entrejambe (13) du slip qui est opposée au corps.

4. Serviette selon l'une quelconque des revendications 1 à 3, caractérisée par le fait qu'elle comporte des éléments d'adhérence (12) pour la fixation du dépassement (6) au côté du slip qui est opposé au corps.

5. Serviette selon l'une quelconque des revendications 1 à 4, caractérisée par le fait que la protection supplémentaire (10) du linge est constituée d'une feuille, par exemple de polyéthylène ou de polypropylène.

6. Serviette selon l'une quelconque des revendications 1 à 5, caractérisée par le fait que la protection supplémentaire (10) du linge est constituée d'une matière composite comprenant une couche non tissée (1) ou similaire, douce et non irritante pour la peau, et une feuille imperméable aux liquides (3).

7. Serviette selon l'une quelconque des revendications 1 à 6, caractérisée par le fait que la protection supplémentaire (10) du linge est constituée d'une couche de recouvrement douce et non irridante pour la peau (1), d'une couche absorbante (2) située au milieu, de préférence de moins de 4 mm d'épaisseur et d'une feuille imperméable aux liquides (3).

8. Serviette hygiénique selon l'une quelconque des revendications 1 à 7, caractérisée par le fait que la protection supplémentaire (10) du linge, pourvue de la surface douce et non irritante pour la peau s'applique, en particulier adhère, au côté de la serviette qui est opposé au corps.

9. Serviette hygiénique selon l'une quelconque des revendications 1 à 8, caractérisée par le fait que le dépassement latéral (6) fait saillie aux extrémités longitudinales (8) de la serviette.

Fig.1

Fig.2

0 127 103

Fig. 3

Fig. 4

8
5
7
9
6

10

8
11
12

0 127 103

Fig. 5

Fig. 6

0 127 103